Europäisches Patentamt

European Patent Office

Office européen des brevets

(11) Veröffentlichungsnummer : **0 023 228**
**B1**

(12) **EUROPÄISCHE PATENTSCHRIFT**

(45) Veröffentlichungstag der Patentschrift :
05.06.85

(51) Int. Cl.⁴ : **A 61 B 17/56**

(21) Anmeldenummer : **79102630.5**

(22) Anmeldetag : **25.07.79**

(54) Kompressionsdübel zur intramedullären Knochenbruchstabilisierung.

(43) Veröffentlichungstag der Anmeldung :
04.02.81 Patentblatt 81/05

(45) Bekanntmachung des Hinweises auf die Patenterteilung : 05.06.85 Patentblatt 85/23

(84) Benannte Vertragsstaaten :
AT CH DE FR GB SE

(56) Entgegenhaltungen :
CH-A- 453 570
DE-A- 2 127 881
DE-A- 2 701 279
DE-B- 1 965 350
DE-B- 2 112 138
DE-B- 2 440 045
FR-A- 1 416 585

(73) Patentinhaber : **Stürmer, Michael, Dr. med.**
**Sundernholz 86**
**D-4300 Essen-Stadtwald (DE)**

(72) Erfinder : **Stürmer, Michael, Dr. med.**
**Sundernholz 86**
**D-4300 Essen-Stadtwald (DE)**

**Beschreibung**

Die Erfindung betrifft eine Vorrichtung zur Stabilisierung von gebrochenen Knochen im Rahmen der operativen Behandlung von Knochenbrüchen (Osteosynthese).

Bei der Osteosynthese von Knochen, insbesondere Röhrenknochen, ist es bekannt, in die Markhöhle, o. h. intramedullär, Marknägel in Form von geraden oder gebogenen Metallstäben zur Stabilisierung der Bruchstücke einzuführen. Es ist ferner bekannt, an Stelle massiver Stäbe mehr oder minder längsgeschlitzte, rohrähnliche Hohlprofile zu verwenden. Darüber hinaus gibt es auch sogenannte, als Hohlprofile ausgeführte « Kompressionsnägel », bei denen mit Hilfe eines zwischen zwei Arretierschrauben gespannten und durch das Hohlprofil geführten Drahtes die Knochenbruchstücke zusammengezogen und gegeneinander verspannt werden können.

Ein Nachteil der normalen Marknägel ist die mangelhafte Ruhigstellung im Bruchspalt bei Dreh- und Biegebelastungen des Knochens. Bei Kompressionsnägeln wird durch das Zusammenpressen der Knochenstücke eine hohe Reibung im Bruchspalt erzeugt und dadurch die Relativbewegung der Bruchstücke gegeneinander weitgehend unterdrückt. Dieses Ziel ist jedoch nur mit einem großen operationstechnischen Aufwand, insbesondere mit einer zusätzlichen Weichteil- und Knocheneröffnung sowie entsprechender zusätzlicher Strahlenbelastung durch laufende Röntgenkontrollen zu erreichen. Außerdem haben Untersuchungen an Leichenknochen gezeigt, daß die über den verhältnismäßig dünnen Draht erzeugte Vorspannung schon durch geringe Biegung des Knochens abgebaut wird. Eine Sicherung gegen Verdrehen der Knochenbruchenden gegeneinander ist jedoch nur bei ausreichend hohem Kompressionsdruck und entsprechender Form der Bruchstücke gewährleistet. Bei ungünstiger Belastung ist hingegen auch bei den bisher bekannten Kompressionsdübeln ein Drehfehler der operierten Knochen mit allen nachteiligen Folgen für den Patienten nicht sicher vermeidbar. Ein weiterer Nachteil der bisher bekannten Kompressionsdübel ist die fehlende Längenverstellbarkeit des Schaftes. So wird eine kostspielige Lagerhaltung verschiedener Nagel- und Dübellängen mit jeweils verschiedenen Durchmessern auch in solchen Krankenhäusern notwendig, in denen selten ein solches Implantat zur Osteosynthese gebraucht wird. Zudem ist die exakte Vorbestimmung der Nagel- oder Dübellänge vor und während der Operation schwierig, so daß gelegentlich ein bereits eingesetzter, zu kurzer oder zu langer Nagel oder Dübel ausgetauscht werden muß.

Aus der DE-A-2 127 881 ist ein Kompressionsdübel gemäß dem Oberbegriff des Anspruchs 1 mit einer durch eine Drehbewegung einer Hülse bewirkten Schaftverlängerung bekannt, wobei aber eine Verdrehungssicherung zwischen Schaft und Hülse nicht möglich ist. Aus der DE-A-2 701 279 ist eine Verdrehungssicherung zwischen Knochen und Profilschaft bei einem Kompressionsdübel bekannt, jedoch ist bei diesem Kompressionsdübel keine Schaftverlängerung vorgesehen.

Ziel der Erfindung ist es, die Nachteile der oben geschilderten Marknägel und Kompressionsdübel zu vermeiden. Dies geschieht erfindungsgemäß mittels eines Kompressionsdübels der bekannten Art, bei dem die Hülse einerseits gegenüber dem Profilschaft und andererseits gegenüber der Knochensubstanz, mit der sie in Berührung steht, gegen Verdrehung gesichert ist. Die beschriebene Erfindung läßt sich ferner noch dadurch verbessern, daß die längsverschiebbare Hülse nicht rotationssymmetrisch gestaltet oder mit Haken oder Dornen versehen ist. Die Haken oder Dornen dieser Hülse treten beim Vorspannen des Dübels in die Knochensubstanz ein und verhindern damit die Verdrehung dieses Knochenbruchstücks gegenüber dem Dübel. Da das andere Ende des Knochens durch den Spreizmechanismus bereits gegen Verdrehung gesichert ist, wird unabhängig von der Längsvorspannung, also auch bei einem allmählichen Nachlassen der bei der Operation aufgebrachten Vorspannung, das Auftreten eines Drehfehlers sicher verhindert. Die längsverschiebbare Hülse erlaubt dabei eine Anpassung des Dübels an die individuelle Knochenlänge, speziell dann, wenn es verschieden lange Ausfertigungen der Hülse gibt. Zudem verhindert die genannte Hülse ein Einwachsen von Knochen in den Einschlagkanal, so daß zur Entfernung des Dübels nach ca. 1-2 Jahren keine Knochensubstanz abgemeißelt werden muß.

Die zur Betätigung des Spreizmechanismus vorgesehene Schraube wird unmittelbar vor dem Spreizmechanimus im Profilschaft geführt und so möglichst kurz gestaltet.

Aus der DE-A-2 112 138, der DE-A-2 127 881 sowie der DE-A-2 701 279 ist ein Kompressionsdübel gemäß dem Oberbegriff des Anspruchs 1 mit einem durch eine Schraube zu betätigendem Spreizkörper bekannt, wobei aber diese Schraube als Gewindestange ausgebildet ist und durch die gesamte Länge des Profilschaftes geführt werden muß, weil ihre Führung jeweils im Kompressionsteil des Dübels angeordnet ist.

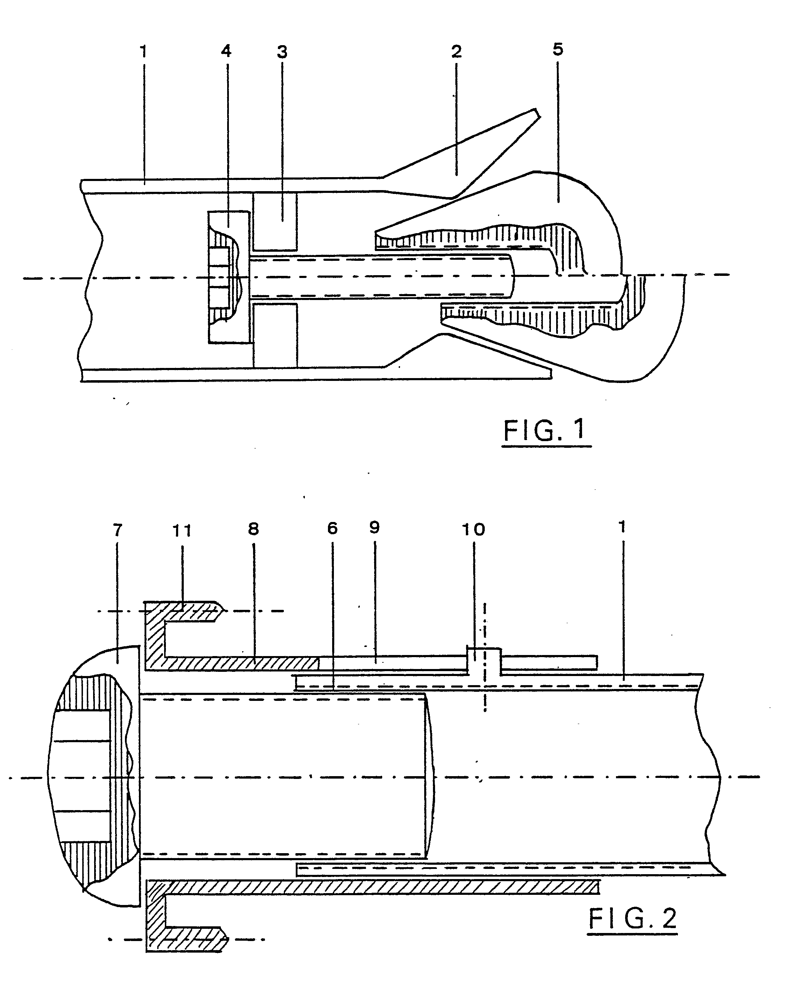

Eine mögliche Ausführung des erfindungsgemäßen Kompressionsdübels ist in den beiliegenden Abbildungen gezeigt wobei Figur 1 die Spreizeinrichtung und Figur 2 die Spanneinrichtung zeigt. Der Profilschaft (1) ist an seiner Spitze mit mehreren Längsschlitzen (2) versehen. Ein ringförmiger Bund (3) dient als Führung für eine Zylinderkopfschraube mit Innensechskant (4). Diese Schraube zieht einen birnenförmigen mit Längsrillen (nicht dargestellt) versehenen Einsatz, Spreizkörper genannt (5), in den geschlitzten Teil des Profilschaftes (1) und spreizt damit

die einzelne Segmente radial auseinander. Das andere Ende des Profilschaftes (1) ist mit einem Innengewinde (6) versehen, welches eine Kugelkopfschraube (7) mit Innensechskant aufnimmt. Eine längsverschiebbar auf dem Profilschaft (1) angeordnete Hülse (8) ist mit einer Nut (9) ausgestattet. In diese Nut greift ein zylindrischer Stift (10) oder eine ähnliche Vorrichtung an der Außenseite des Profilschaftes (1) ein und verhindert so ein Verdrehen der Hülse (8) gegenüber dem Profilschaft (1). Der scheibenförmige Teil der Hülse (8) ist mit dornenförmigen Fortsätzen (11) ausgerüstet.

Die Entfernung des Kompressionsdübels erfolgt zunächst durch Lösen und Herausnehmen der Schraube (7). Sodann wird die Schraube (4) gelöst und damit die innere Abstützung der gespreizten Segmente aufgehoben. Nun wird in das Innengewinde (6) des Profilschaftes (1) eine Stange eingeschraubt und der Dübel mit einem Schlaggewicht aus dem Knochen heraus geschlagen. Dabei wird die Spreizung der Segmente problemlos aufgehoben, die Segmente legen sich wieder an. Wegen der geschlossenen Bauweise des Dübels ist ein Einwachsen von Knochensubstanz und eine dadurch bedingte Behinderung der Dübelentfernung nicht möglich.

Ein erfindungsgemäßer Kompressionsdübel konnte an der quer durchtrennten Tibia von Schafen experimentell erfolgreich erprobt werden. Die Knochenfragmente kamen unter hohe Kompression und blieben postoperativ drehstabil fixiert, obwohl es sich um glatte Querosteotomien handelte und die Tiere voll belastend herumliefen. Die knöcherne Überbrückung der Osteotomie erfolgte bereits nach einem Monat, wie die röntgenologische und histologische Untersuchung zeigte.

## Patentansprüche

1. Kompressionsdübel zur Stabilisierung von gebrochenen Knochen, vorzugsweise Röhrenknochen, durch Einführen eines rohrähnlichen hohlen Profilschaftes in die Markhöhle, wobei der Profilschaft an seinem einen Ende eine in radialer Richtung wirkende Spreizeinrichtung aufweist mit einem Spreizkörper (5), der mittels einer Schraube (4) vom anderen Schaftende aus in das längsgeschlitzte Spreizende des Profilschaftes gezogen werden kann und dabei die Segmente desselben auseinander spreizt, ferner mit einer von der Spreizeinrichtung unabhängigen, zur Längsspannung dienenden, am anderen Ende des Kompressionsdübels vorgesehenen, eine axiale Schaftlängenänderung bewirkenden Hülse (8), dadurch gekennzeichnet, daß die Hülse (8) in axialer Richtung auf dem Profilschaft gleitet und daß die Hülse einerseits gegenüber dem Profilschaft und andererseits gegenüber der Knochensubstanz, mit der sie in Berührung steht, gegen Verdrehung gesichert ist.

2. Kompressionsdübel nach Anspruch 1, dadurch gekennzeichnet, daß das Spreizende bei gleichbleibendem Außendurchmesser dickwandiger gestaltet wird als der übrige Schaft.

3. Kompressionsdübel nach Anspruch 1, dadurch gekennzeichnet, daß die Hülse in dem mit der Knochensubstanz in Berührung stehenden Teil nicht rotationssymmetrisch ausgebildet ist.

4. Kompressionsdübel nach Anspruch 1, dadurch gekennzeichnet, daß die Hülse dornenähnliche, sich in axialer Richtung erstreckende Fortsätze im Bereich des äußeren Umfangs trägt.

5. Kompressionsdübel nach Anspruch 1, dadurch gekennzeichnet, daß die zum Spreizen vorgesehene Schraube (4) unmittelbar vor dem Spreizende des Profilschaftes angeordnet ist und auch nur dort ihre Führung (3) hat, so daß der Mittelteil und der Kompressionsteil des Dübels unabhängig vom Spreizmechanismus gestaltet werden können.

6. Kompressionsdübel nach Anspruch 5, dadurch gekennzeichnet, daß die Schraube (4) in der Führung (3) gelagert ist und ihr Gewinde im Spreizkörper faßt.

## Claims

1. Compression peg for stabilisation of broken bones, especially long bones, through insertion of a tube type profile shaft into the marrow cavity, whereby the profile shaft shows on one end in its radial direction a spreadfitting (5), which may be adjusted from the other end of the profile shaft through the axial slit spread end by means of a screw (4) and thereby to spread the segments of the shaft, furthermore it contains independend speadfitting to maintain tension at the other end of the compression peg by way of a shaft length changing shell (8), identified by the shell (8) gliding in axial direction on the profile shaft and that the shell secures on one hand the profile shaft and on the other hand the bone substance, with which it is in contact, against twisting.

2. Compression peg according to claim 1 is caracterised by the spread end being shaped thicker than the remaining shaft, although the outer diameter remaines the same.

3. Compression peg according to claim 1 identified by the shell not being rotation symmetrical in the part, which is in contact with the bone substance.

4. Compression peg according to claim 1 identified by the shell containing thorn like continuations, which are carried in axial direction within the outer perimeter.

5. Compression peg according to claim 1 identified by the fact, that the screw intended for spreading (4) is located immediately in front of the spread end of the profile shaft and only there it has it's guidance (3), so that the center part and the compression part of the peg are independend from the spread mechanism.

6. Compression peg according to claim 5 identified, that the screw (4) is situated in a guidance mechanism (3) and the thread is located in the spread body.

**Revendications**

1. Cheville de compression servant à stabiliser des os fracturés, de préférence des os tubulaires, moyennant l'introduction d'une tige profilée creuse en forme de tube dans la cavité médullaire, la tige profilée comportant sur l'une de ses extrémités un dispositif écarteur s'étendant suivant la direction radiale et muni d'un corps d'écartement (5) qui peut être rétracté, au moyen d'une vis (4), dans l'extrémité d'écartement fendue longitudinalement de la tige profilée, à partir de l'autre extrémité de la tige, et qui écarte les uns des autres les segments de cette tige, et comportant en outre une douille (8) servant à la mise en tension longitudinale, prévue sur l'autre extrémité de la cheville de compression et provoquant une modification de la longueur axiale de la tige, caractérisée en ce que la douille (8) glisse suivant la direction axiale sur la tige profilée et que la douille est bloquée contre toute rotation d'une part par rapport à la tige profilée et d'autre part par rapport à la substance osseuse, avec laquelle elle est en contact.

2. Cheville de compression suivant la revendication 1, caractérisée par le fait que l'extrémité d'écartement est réalisée avec une paroi plus épaisse que le reste de la tige, pour un diamètre extérieur restant constant.

3. Cheville de compression selon la revendication 1, caractérisée en ce que la douille n'est pas réalisée avec une symétrie de révolution dans la partie en contact avec la substance osseuse.

4. Cheville de compression selon la revendication 1, caractérisée en ce que la douille porte, dans les régions de son pourtour extérieur, des appendices saillants en forme d'ergots, s'étendant suivant la direction axiale.

5. Cheville de compression selon la revendication 1, caractérisée en ce que la vis (4) prévue pour effectuer l'écartement est disposée directement en avant de l'extrémité d'écartement de la tige profilée et que son guide (3) n'est également présent qu'en cet emplacement, de sorte que la partie médiane et la partie de compression de la cheville peuvent être conformées d'une manière indépendante du mécanisme écarteur.

6. Cheville de compression selon la revendication 5, caractérisée en ce que la vis (4) est montée dans le guide (3) et engrène par son filetage dans le corps d'écartement.

FIG. 1

FIG. 2